# EUROPEAN PATENT APPLICATION

(11) **EP 1 447 975 A2**
(43) Date of publication of application: **18.08.2004**
(21) Application number: 04250757.4
(22) Date of filing: 12.02.2004
(51) Int. Cl.: H04N 3/233

(54) **Projected image correction method and projector**

(30) Priority: 17.02.2003 JP 2003038050
(71) Applicant: SEIKO EPSON CORPORATION, Shinjuku-ku, Tokyo 163-0811 (JP)
(72) Inventor: Nashida, Yukihiro, c/o Seiko Epson Corp., Suwa-shi Nagano-ken 392-8502 (JP); Nagano, Miki, c/o Seiko Epson Corp., Suwa-shi Nagano-ken 392-8502 (JP); Kamiya, Yasutaka, c/o Seiko Epson Corp., Suwa-shi Nagano-ken 392-8502 (JP); Takei, Akira, c/o Seiko Epson Corp., Suwa-shi Nagano-ken 392-8502 (JP); Yoshikuni, Norihiro, c/o Seiko Epson Corp., Suwa-shi Nagano-ken 392-8502 (JP)
(74) Representative: Sturt, Clifford Mark

(57) **Abstract**

The present invention aims to execute a projected image correction method and a projector that can execute keystone correction of larger amount without modifying or replacing a keystone correction device. A projector according to the invention includes a signal scaling down device 14 that scales down image signals that are input, a keystone correction device 15 that executes keystone correction for the scaled down image signals, a buffer 16 that temporarily stores the image signals for keystone correction, and a signal interpolation device 17 that interpolates the corrected image signals.

## Description

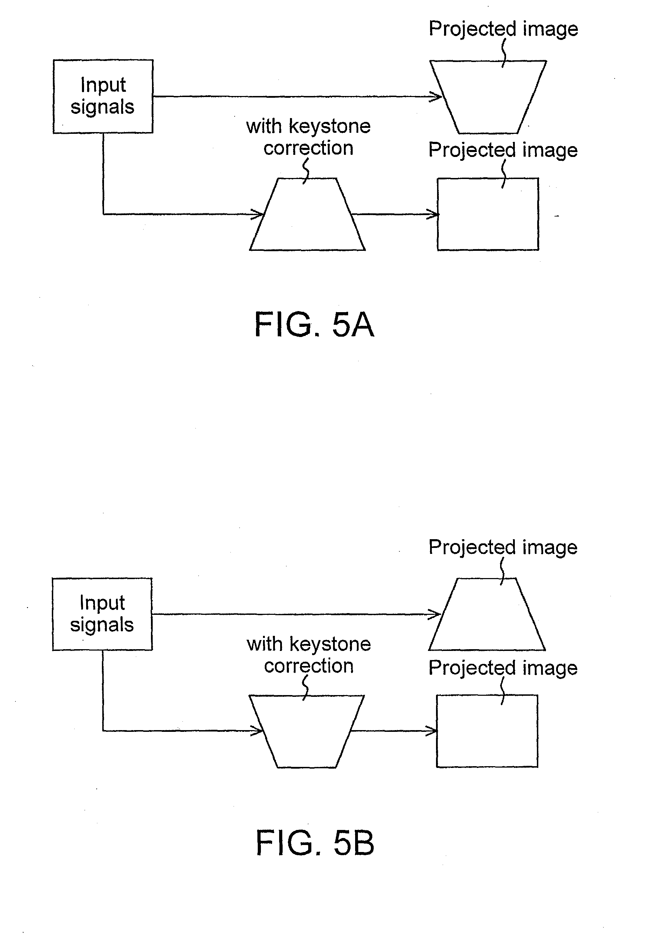

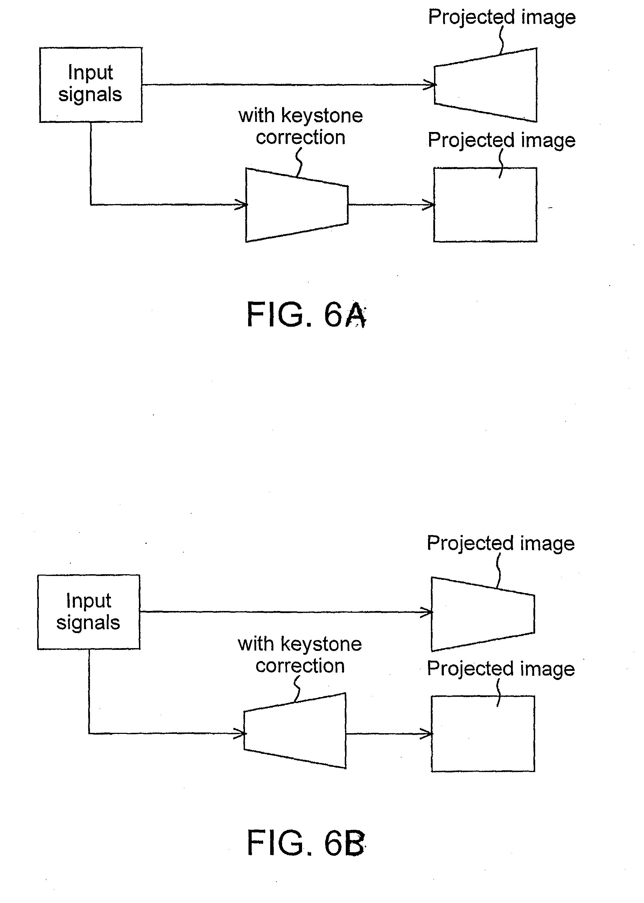

The present invention relates to a projected image correction method and a projector, and more particularly to keystone correction. A projector expands an original image for display that is output from an information processor such as a computer, and projects the expanded image on a projection surface such as a screen. If the image is projected on the screen surface under the state that the central axis of the projected image is tilted from the projection axis of a screen, the projected image is distorted on the screen. Specifically, if a rectangular image is projected on the screen by having the projector above or below the center of the screen surface, the image appears in the shape of a trapezoid on the screen (described later in greater detail with reference to FIGs. 5 and 6).

To solve this problem, a method is used to adjust for the phenomenon that an image that is different from its original image is projected and to display a projected image almost the same of the original image without distortion. This is called keystone correction. Various methods have been developed to execute keystone correction, for example, as described in the related art document. In order to execute keystone correction, a projector is equipped with a keystone correction device.

### [Related Art Document]

Japanese Unexamined Patent Application Publication No. 2002-6391.

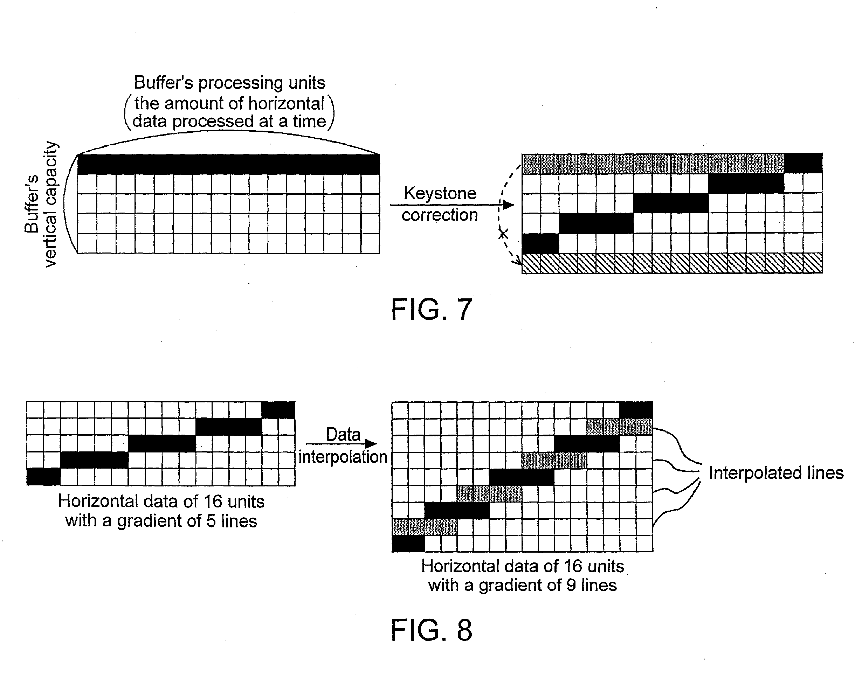

The keystone correction device included in a projector has a buffer where input signals (image signals) for correction are stored temporarily. However, horizontal keystone correction involves the following problem. Since it is necessary to store data of the number of lines according to the degree of a tilted angle, the amount of image correction is restricted due to the capacity of the buffer (described later in greater detail with reference to FIG. 7). Therefore, in order to execute image correction beyond the capacity, the keystone correction device requires to be modified or replaced.

In order to solve the problem, the invention aims to execute a projected image correction method and a projector that can execute keystone correction of larger amount without modifying or replacing the keystone correction device.

A projected image correction method according to the present invention includes the following steps: scaling down image signals that are input, executing keystone correction for the scaled down image signals, and interpolating the corrected image signals. According to the invention, the image signals are scaled down before keystone correction to reduce the amount of signals to be corrected. Then the corrected image signals are interpolated to compensate for the signal scale down so as to reproduce the original image signals. This makes it possible to execute keystone correction of larger amount without changing keystone correction devices, for example.

The projected image correction method according to the invention executes at least horizontal keystone correction in the step of executing keystone correction. When executing horizontal keystone correction, for example, the amount of image correction depends on the capacity of a buffer for a keystone correction device (that is, the amount of image correction is limited by the buffer capacity). The invention solves this problem by scaling down the image signals before keystone correction to reduce the amount of signals to be corrected, and interpolating the corrected signals to compensate for the signal scale down so as to reproduce the original image signals. This makes it possible to execute horizontal keystone correction of larger amount.

The projected image correction method according to the invention vertically scales down the image signals at predetermined intervals in the step of scaling down the image signals. When executing horizontal keystone correction, the capacity of a buffer for a keystone correction device needs to be increased. However, by vertically scaling down the image signals at predetermined intervals, it is sufficient to have a keystone correction device with a buffer of small capacity. This makes it possible to execute image correction of larger amount under the condition that the buffer has a fixed capacity.

The projected image correction method according to the invention also vertically scales down the image signals according to a function that is preset. Scaling down the signals according to the type of image, for example, enables efficient signal scale down.

The projected image correction method according to the invention synthesizes data on scaled down lines with data on non-scaled down lines in the step of scaling down the image signals. This improves data reliability for signal interpolation.

The projected image correction method according to the invention interpolates the image signals in response to the step of scaling down the image signals.

A projector according to the invention includes a signal scaling down means that scales down image signals that are input, a keystone correction means that executes keystone correction for the scaled down image signals, a storage means that temporarily stores the image signals for keystone correction, and a signal interpolation means that interpolates the corrected image signals. According to the invention, the image signals are scaled down by the signal scaling down means before the keystone correction means executes keystone correction. This reduces the amount of signals to be corrected by the keystone correction means. Then the corrected image signals are interpolated in order to compensate for the signal scale down so as to reproduce the original image signals. Consequently, it is possible to execute keystone correction of larger amount without changing the capacity of the storage means (a buffer).

The keystone correction means of the projector according to the invention executes at least horizontal keystone correction. When executing horizontal keystone correction, for example, the amount of image correction is limited to the capacity of the buffer for the keystone correction means. The invention solves this problem by scaling down the image signals before keystone correction. Accordingly, the invention makes it possible to execute horizontal keystone correction of larger amount.

The signal scaling down means of the projector according to the invention vertically scales down the image signals at predetermined intervals. When executing horizontal keystone correction, the capacity of the storage means (buffer) for keystone correction needs to be increased as mentioned above. However, by vertically scaling down the image signals at predetermined intervals, it is sufficient to have the storage means (buffer) of small capacity. This makes it possible to execute image correction of larger amount under the condition that the storage means (buffer) has a fixed capacity.

The signal scaling down means of the projector according to the invention also vertically scales down the image signals according to a function that is preset.

The signal scale down means of the projector according to the invention synthesizes data on scaled down lines with data on non-scaled down lines.

The signal interpolation means of the projector according to the invention interpolates the image signals in response to the step of scale down the image signals by the signal scale down means.

The projector according to the invention also includes a controller. The amount of keystone correction that is executed by the keystone correction means is determined based on operational signals that are sent by the controller.

Embodiments of the present invention will now be described by way of further example only and with reference to the accompanying drawings, in which:-
FIG. 1 is a block diagram showing the configuration of a projector according to a first embodiment of the invention.
FIG. 2 is a flowchart showing the process of data processing of the projector shown in FIG. 1.
FIG. 3 illustrates data scale down.
FIG. 4 illustrates data scale down.
FIG. 5 illustrates vertical keystone correction.
FIG. 6 illustrates horizontal keystone correction.
FIG. 7 illustrates how a buffer works for keystone correction.
FIG. 8 illustrates data interpolation.
FIG. 9 is a block diagram showing the configuration of a projector according to a second embodiment of the invention.

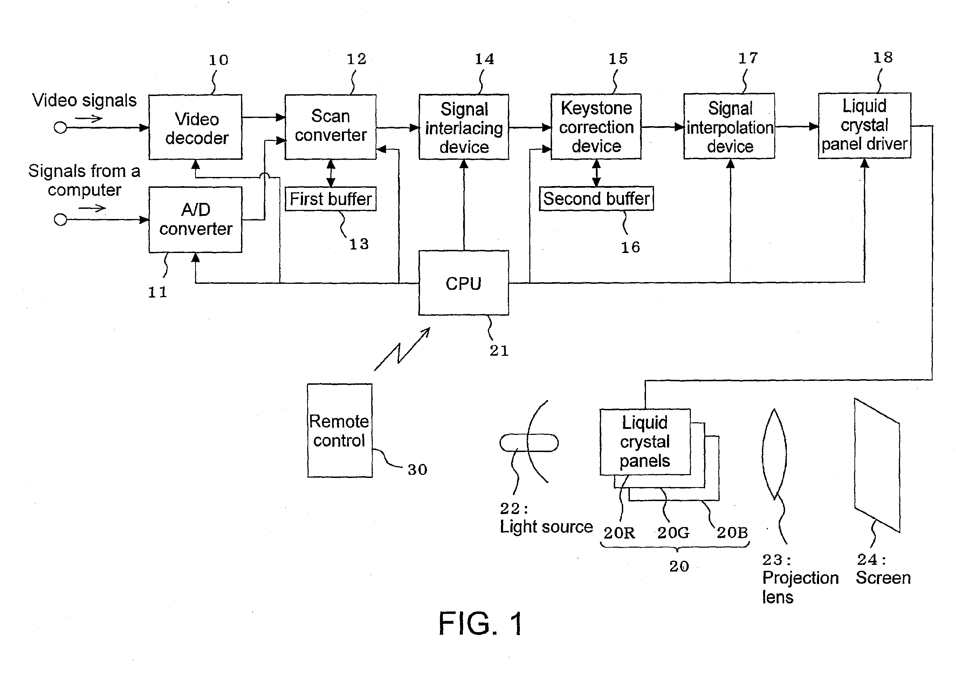

FIG. 1 is a block diagram showing the configuration of a projector according to a first embodiment of the invention. The diagram shows an example of individual circuit blocks from an input signal part such as a video decoder to a liquid crystal panel driver as a final stage are each formed as an integrated circuit. The projector includes a video decoder 10, an A/D converter 11, a scan converter 12, a first buffer 13, a signal scaling down device 14, a keystone correction device 15, a second buffer 16, a signal interpolation device 17, a driver for driving liquid crystal panels (a liquid crystal panel driver) 18, liquid crystal panels 20, and a central processing unit (CPU) 21. The projector also includes a light source 22 and a projection lens 23. It should be noted that FIG. 1 shows circuits components of the projector that are necessary to describe the first embodiment and omits other circuit components.

The video decoder 10 is coupled to the scan converter 12 at its output terminal. The video decoder 10 receives video signals (composite signals, S signals, etc.) output by a video device, decodes and digitizes the signals, and outputs the digitized signals to the scan converter 12. The A/D converter 11 is also coupled to the scan converter 12 at its output terminal. The A/D converter 11 receives image signals (VGA, XGA, SXGA, etc.) output by a computer, digitizes the signals, and outputs the digitized signals to the scan converter 12. The scan converter 12 is coupled to both the first buffer 13 and the signal scaling down device 14. The scan converter 12 converts frame rates of the signals with the first buffer 13, and outputs the signals of which frame rate is changed to the signal scaling down device 14.

The signal scaling down device 14 is coupled to the keystone correction device 15 at its output terminal. The signal scaling down device 14 scales down the signals output by the scan converter 12 and outputs the scaled down signals to the keystone correction device 15. The keystone correction device 15 is coupled to both the second buffer 16 and the signal interpolation device 17. The keystone correction device 15 executes keystone correction for the signals scaled down and output by the signal scaling down device 14 with the second buffer 16.

The signal interpolation device 17 is coupled to the liquid crystal panel driver 18 at its output terminal. The signal interpolation device 17 interpolates the signals scaled down by the signal scaling down device 14 and outputs the interpolated signals to the liquid crystal panel driver 18. The liquid crystal panel driver 18 drives the liquid crystal panels 20. The liquid crystal panels 20 include three panels, for example, a red panel 20R, a green panel 20G, and a blue panel 20B. Each of the panels is driven to produce an image.

When the liquid crystal panels 20 (20R, 20G, and 20B) are driven by the liquid crystal panel driver 18, the transmittance of liquid crystal is controlled in response to the image signals. The transmittance of light from the light source 22 such as a lamp is thus controlled to produce an image. The image produced by-the liquid crystal panels 20 (20R, 20G, and 20B) is expanded and projected on a screen 24 through the projection lens 23.

The CPU 21 controls the whole of the components of the projector. In this embodiment, the CPU 21 controls, based on operational signals sent by a remote control 30, the video decoder 10, the A/D converter 11, the scan converter 12, the signal scaling down device 14, the keystone correction device 15, the signal interpolation device 17, and the liquid crystal panel driver 18. Also in this embodiment, the CPU 21 decides the amount of image correction, executed by the keystone correction device 15, based on operational signals sent from the remote control 30.

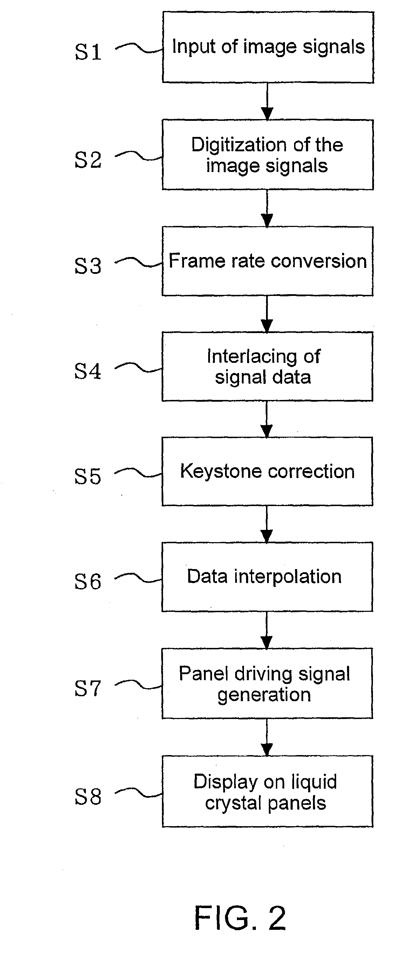

FIG. 2 is a flowchart showing the process of data processing of the projector shown in FIG. 1. With reference to this flowchart, the process that is handled by each of the components shown in FIG. 1 is described below.

### (S1) Input of image signals

The video decoder 10 and the A/D converter 11 are each coupled to an external device such as a video device and a computer and receive signals from them. TABLE 1 shows examples of external video devices that are coupled and of video signals. TABLE 2 shows the types of the signals of various resolution levels and refresh rates.

**TABLE 1**

| External device | Signal |
|---|---|
| Video device | Composite / S-signal / Component (color difference signal) |
| DVD device | Composite / S-signal / Component (color difference signal) |

**TABLE 2**

| Signal | Type |
|---|---|
| Composite | NTSC, PAL, SECAM, etc. |
| S | |
| Component (color difference signal) | 525i, 525p, 625i, 625p, 750p, 1125i, 1125p, etc. |

TABLE 3 shows examples of external computer devices that are coupled and of these signals. TABLE 4 shows the types of the signals of various resolution levels and refresh rates.

**TABLE 3**

| External device | Signal |
|---|---|
| Personal Computer | Analog RGB |
| High definition decoder | Analog RGB / Color difference signal |
| Video-to-PC converter | Analog RGB |

**TABLE 4**

| Signal | Type |
|---|---|
| Analog RGB | Resolution level: VGA, SVGA, XGA, SXGA, etc. |
| | Refresh rate: 60 Hz, 75 Hz, 85 Hz, etc. |
| | Interlace / Non-interlace |
| Color difference signal | 525i, 525p, 625i, 625p, 750p, 1125i, 1125p, etc. |

### (S2) Digitization of the image signals

The signals that are input in the process S1 are analog image signals. The analog signals are then digitized, and thereby can be handled by the scan converter 12 later in the process. If the scan converter 12 handles analog inputs instead of digital inputs, this digitization stage can be omitted. Here, the analog video signals are decoded by the video decoder 10 and digitized. The signals from a computer are decoded by the A/D converter 11 and digitized. The types of output signals vary according to the specifications of the video decoder 10 and the A/D converter 11. TABLE 5 shows the general types of the signals.

**TABLE 5**

| Signal line | Details |
|---|---|
| Clock signal | In order to enable picture signal data to be normally input, a single clock signal is produced corresponding to single picture signal data. |
| Vertical sync signal (VSync) | This signal is produced corresponding to data of single image display. Refresh rate is the frequency of this signal. The refresh rate of input signal is equivalent to the frequency. |
| Horizontal sync signal (HSync) | This signal is produced every data on a single line. |
| Image signal data | This signal is obtained by digitizing an analog voltage signal. Normally composed of eight to ten signal lines each of R, G, and B. |
| Data enable signal | This signal indicates the validity or invalidity of image signal data. |

### (S3) Frame rate conversion

The size and refresh rate of the digital image signals that are output by the video decoder 10 and the A/D converter 11 vary according to the types of the signals. The scan converter 12 converts such size and refresh rate of the digital image signals into the predetermined size and refresh rate of it. The predetermined size and refresh rate of the signals output from the scan converter 12 are determined by input conditions and specification of the signal scaling down device 14 on the downstream side and other devices to which the signals are to be input afterward. Furthermore, the predetermined size and refresh rate are controlled by the CPU 21. The signals that are output by the scan converter 12 are in the same format as the digital image signals output by the video decoder 10 etc. However, the frequency, size, and so on of the signals are different.

### (S4) Scale down of signal data

The signal scaling down device 14 scales down the digital image signals that are input by the scan converter 12 at predetermined intervals (can be nonlinear) and outputs the scaled down digital image signals. More specifically, the signal scaling down device 14 scales down horizontal and vertical synchronizing signals or data enable signals among the signals that are output by the scan converter 12 at predetermined intervals (can be nonlinear) to scale them and outputs the scaled down image signals to the keystone correction device 15 on the downstream side. Consequently, the keystone correction device 15 receives not all the image signals that are output by the scan converter 12 but the scaled down image signals part of which are scaled down as input signals.

Methods for scaling down data by means of the signal scaling down device 14 may include but are not limited to the following:
(a) Scaling down data lines at regular intervals, for example, a line for every n lines or m lines for every n lines ("n" is larger than "m", e.g., 2 lines for every 3 lines).
(b) Scaling down data lines according to a function that is preset in the signal scaling down device 14. (nonlinear)
(c) Scaling down data lines horizontally and vertically in the same manner or in different manner.
(d) Synthesizing data on scaled down data lines with data on non-scaled down data lines so as to retain the whole data.

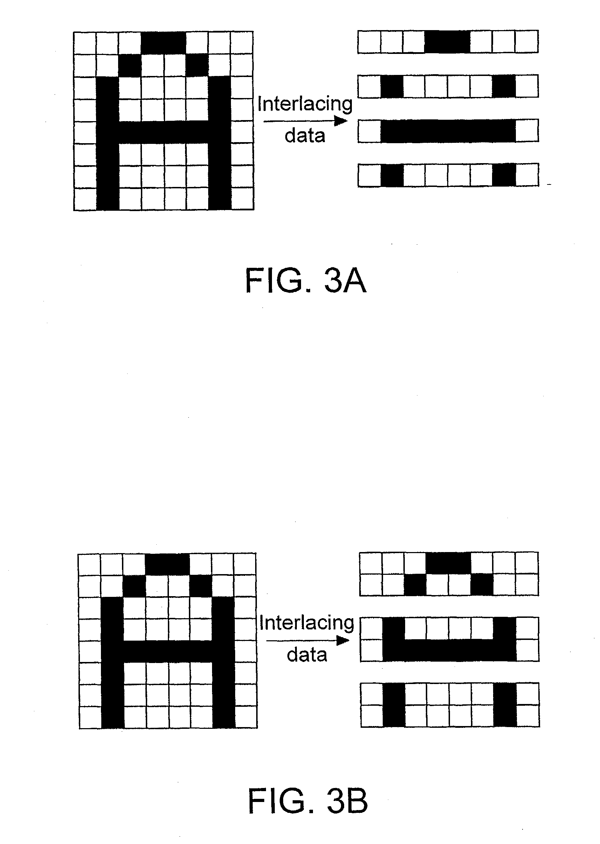

FIGs. 3 (A) and (B) show examples of data scale down by the signal scaling down device 14. FIG. 3 (A) shows an example of scaling down a data line for every two input lines, while FIG. 3 (B) shows an example of scaling down a data line for every three input lines.

The data on scaled down data lines may be synthesized with non-scaled down data lines.

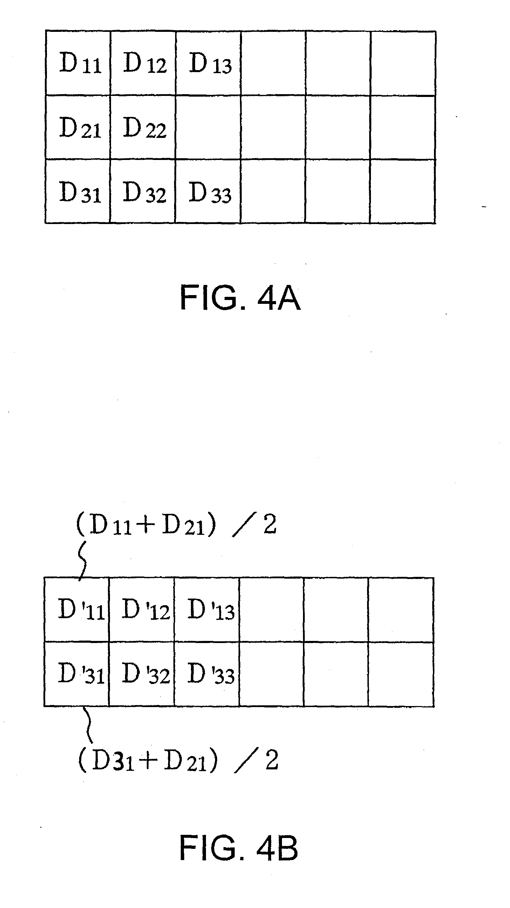

FIG. 4 shows an example of data scale down. In this example, the data of the data line L2 (D₂₁, D₂₂, ...) among the three data lines shown in FIG. 4 (A) are scaled down. As FIG. 4 (B) shows, the data of the line L2 are synthesized with the data of the lines L1 and L3. Thus the data that were scaled down are obtained as a synthesis of the data of the lines L1 and L3. For example, the data of the pixel D'₁₁ of the data line L1 is obtained by the formula: (D₁₁ + D₂₁) / 2. The data of other pixels can be obtained in the same way.

### (S5) Keystone correction

The keystone correction device 15 executes keystone correction for the image signals that are output by the signal scaling down device 14 and outputs the corrected signals to the signal interpolation device 17. Keystone correction generally includes horizontal correction and vertical correction. A hybrid of the both is also available. The keystone correction device 15 executes keystone correction by nonlinear compressing and nonlinear converting horizontal and vertical data of input signals. This requires the second buffer 16 that performs operations for data compression. Vertical correction, which involves horizontal data compression, requires only data areas corresponding to horizontal data lines. Meanwhile, horizontal keystone correction, which involves compression with a vertical gradient, requires a buffer of which capacity has to cover all of data lines along compressed vertical direction. As a result, the gradient that can be corrected is restrained by the capacity of the second buffer 16. (See FIG. 7.)

FIGS. 5 (A) and (B) illustrate an example of vertical keystone correction. In one case as shown in FIG. 5 (A), although the original shape of input signals (image data) is a rectangle, the image projected on the screen appears in the shape of a trapezoid with a shorter base without keystone correction. This is because vertical lines of the image data are tilted due to the light axis of the projector directed toward the screen 24 at an angle. In this case, the projected image can be a rectangle by altering the shape that is formed by the input signals (image data) to form a trapezoid with a longer base. In another case as shown in FIG. 5 (B), the image projected on the screen appears in the shape of a trapezoid with a longer base without keystone correction. In this case, the projected image can be a rectangle by altering the shape that is formed by the input signals (image data) to form a trapezoid with a shorter base.

FIGs. 6 (A) and (B) illustrate an example of horizontal keystone correction. In one case as shown in FIG. 6 (A), although the original shape of input signals (image data) is a rectangle, the image projected on the screen appears in the shape of a sideways-facing trapezoid whose shorter base is on the left side without keystone correction. This is because horizontal lines of the image data are tilted due to the light axis of the projector directed toward the screen 24 at an angle. In this case, the projected image can be a rectangle by altering the shape that is formed by the input signals (image data) to form a trapezoid with a longer base on the left side. In another case as shown in FIG. 6 (B), the image projected on the screen appears in the shape of a sideways-facing trapezoid whose shorter base is on the right side without keystone correction. In this case, the projected image can be a rectangle by altering the shape that is formed by the input signals (image data) to form a trapezoid with a longer base on the right side.

FIG. 7 illustrates an example of horizontal keystone correction in relation to the capacity of the second buffer 16. In this example, the second buffer 16 has a horizontal capacity equivalent to processing units of the keystone correction device 15 (the amount of horizontal data that can be processed at a time, e.g., horizontal data of sixteen (16) units in the diagram) and a vertical capacity of the predetermined number of data lines (e.g. five (5) data lines in the diagram). This means that the second buffer 16 enables the device to execute keystone correction for horizontal data of up to sixteen (16) units with a vertical gradient of five (5) lines. Therefore, it is impossible to execute keystone correction beyond the capacity, that is, correction for horizontal data of up to sixteen (16) units with a vertical gradient of six (6) lines or more. However, the first embodiment solves this problem by scaling down data with the signal scale down device 14 before entering data into the keystone correction device 15. When scaling down a data line for every two lines as shown in FIG. 3 (A), for example, it is possible to execute keystone correction for horizontal data of up to sixteen (16) units with a vertical gradient of ten (10) lines by performing data interpolation that is described in detail below. For another example, when scaling down a data line for every three lines as shown in FIG. 3 (B), it is possible to execute keystone correction for horizontal data of up to sixteen (16) units with a vertical gradient of seven (7) lines by performing data interpolation as described below.

### (S6) Data interpolation

The signal interpolation device 17 interpolates the signals that are output by the keystone correction device 15 and outputs the interpolated signals to the liquid crystal panel driver 18. The signals output by the keystone correction device 15 lack a predetermined part of data excluded by the signal scaling down device 14. According to this embodiment, the excluded signals are interpolated here before being output to the liquid crystal panel driver 18. As the signals are interpolated by the signal interpolation device 17, data sets increase vertically (that is, the number of data lines increases). In other words, the number of output data lines that form a gradient increases. A variety of interpolation methods are available depending on the type of IC for interpolation. Conventionally without data scale down before keystone correction, a gradient that can be corrected is limited by the capacity of the second buffer 16 for the keystone correction device 15. By scaling down data before executing keystone correction and then interpolating the scaled down data, the number of horizontal data lines that form a gradient can be increased without changing the capacity of the second buffer 16.

FIG. 8 illustrates an example of data interpolation by the signal interpolation device 17. Here, data interpolation is executed in response to the step of scaling down the image signals by the signal scaling down device 14. In this example, signals are scaled down by the signal scaling down device 14 as shown in FIG. 3 (A). As a result of keystone correction by the keystone correction device 15, the signals include data having horizontal data of sixteen (16) units with a vertical gradient of five (5) lines as shown in FIG. 8 (A). In this case, data interpolation is executed by inserting a data line between every two (2) lines of the five (5) lines. Accordingly, the data include horizontal data of sixteen (16) units with a vertical gradient of nine (9) lines.

### (S7) Production of a signal for driving a panel

The liquid crystal panel driver 18 produces signals for driving or controlling liquid crystal panels based on the signals that are interpolated by the signal interpolation device 17, and drives or controls the liquid crystal panels 20 with the produced signals.

### (S8) Display on the liquid crystal panels

When the liquid crystal panels 20 (20R, 20G, and 20B) are driven by the liquid crystal panel driver 18, the transmittance of liquid crystal is controlled according to the image data. The transmittance of light from the light source 22 such as a lamp is thus controlled to produce an image. Accordingly, the image produced by the liquid crystal panels 20 (20R, 20G, and 20B) is expanded and projected on the screen 24 through the projection lens 23. The projected image is a rectangle with keystone correction. In particular, the first embodiment executes a way to adjust for the fact that the light axis of the projection lens 23 is tilted from the screen 24, since a gradient formed by horizontal data lines for keystone correction is increased.

According to the embodiment, the process includes the following steps: firstly scaling down image signals by the signal scaling down device 14, secondly executing keystone correction by the keystone correction device 15, thirdly interpolating the signals that are output by the keystone correction device 15 by the signal interpolation device 17, and then outputting the signals to the liquid crystal panel driver 18. With this process, the first embodiment executes a method for executing keystone correction by which the number of horizontal data lines that form a gradient can be increased without changing the capacity of the second buffer 16.

### Second Embodiment

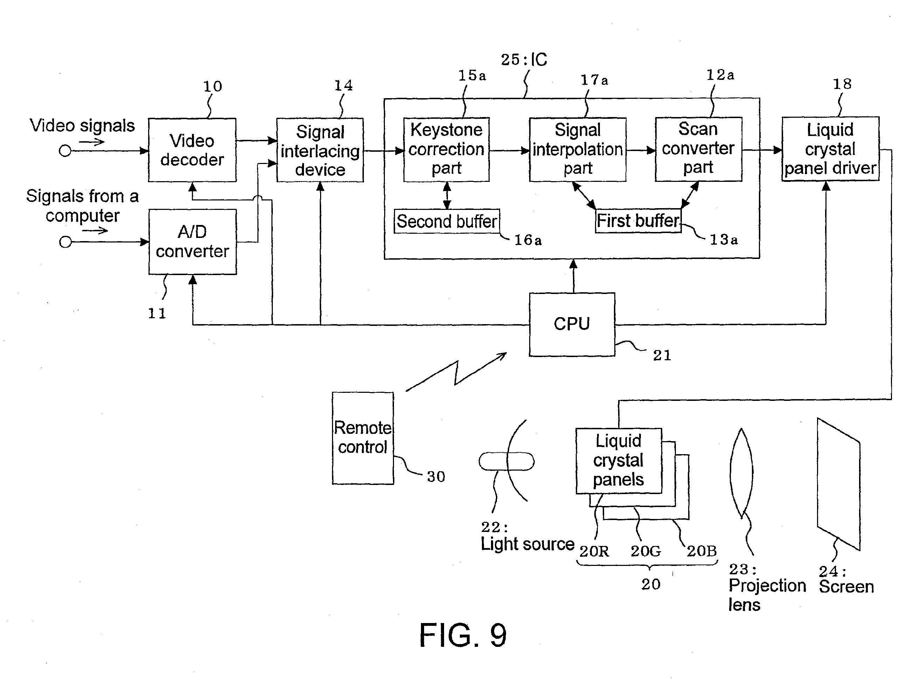

FIG. 9 is a diagram showing the configuration of a projector according to a second embodiment of the invention. The projector according to the second embodiment includes an IC 25 where a keystone correction part 15a, a signal interpolation part 17a, a scan converter part 12a, a first buffer 13a, and a second buffer 16a are integrated.

According to the second embodiment, signals are digitized by the video decoder 10 or the A/D converter 11, scaled down by the signal scaling down device 14, and then output to the IC 25. A method for scaling down the signals by the signal scaling down device 14 is the same as the method described above in the first embodiment. The keystone correction part 15a, which is included in the IC 25, executes keystone correction to the scaled down signals and outputs the corrected signals to the signal interpolation part 17a.

Here, the amount of keystone correction is determined based on the amount of operations that are directed to the CPU 21 by the remote control 30. The CPU 21 then directs the amount of correction to the keystone correction part 15a. The signal interpolation part 17a interpolates the corrected signals and outputs the interpolated signals to the scan converter part 12a. The scan converter part 12a converts frame rates of the signals and outputs the signals to the liquid crystal panel driver 18. The liquid crystal panel driver 18 drives the liquid crystal panels 20. Then the transmittance of liquid crystal is controlled according to the image data. The transmittance of light from the light source 22 such as a lamp is thus controlled to produce an image. The image produced by the liquid crystal panels 20 (20R, 20G, and 20B) is expanded and projected on the screen 24 through the projection lens 23. The projected image is a rectangle with keystone correction as described above.

According to the second embodiment, the IC 25 includes the second buffer 16a for the keystone correction part. Since the capacity of the second buffer 16a is not adjustable, the amount of keystone correction is limited by the capacity of the second buffer 16a. Despite this, the amount of correction can be increased by executing keystone correction after the signal scaling down device 14 scales down the signals, which compensates for the limitation.

### Third Embodiment

In the first embodiment, the example in which the amount of keystone correction is directed by the remote control 30 is cited. Alternatively, the amount of keystone correction can be determined based on a detected degree of angle of a projector body.

## Claims

1. A projected image correction method, comprising:
scaling down image signals that are input;
executing keystone correction for the scaled down image signals; and
interpolating the corrected image signals.

2. The projected image correction method according to Claim 1,
wherein the step of executing keystone correction executes at least horizontal keystone correction.

3. The projected image correction method according to Claim 1 or 2,
wherein the step of scaling down the image signals vertically scales down the image signals at predetermined intervals.

4. The projected image correction method according to Claim 1 or 2,
wherein the step of scaling down the image signals vertically scales down the image signals according to a function that is preset.

5. The projected image correction method according to Claim 3 or 4,
wherein the step of scaling down the image signals synthesizes image data on scaled down lines with data on non-scaled down lines.

6. The projected image correction method according to any one of Claims 1 or 2,
wherein the step of interpolating the corrected image signals interpolates the image signals in response to the step of scaling down the image signals.

7. A projector, comprising:
a means for scaling down image signals that are input;
a means for executing keystone correction for the scaled down image signals;
a means for temporarily storing the image signals for keystone correction; and
a means for interpolating the corrected image signals.

8. The projector according to Claim 7,
wherein the means for executing keystone correction executes at least horizontal keystone correction.

9. The projector according to Claim 7 or 8,
wherein the means for scaling down the image signals vertically scales down the image signals at predetermined intervals.

10. The projector according to Claim 7 or 8,
wherein the means for scaling down the image signals vertically scales downs the image signals according to a function that is preset.

11. The projector according to any one of Claims 9 or 10,
wherein the means for scaling down the image signals synthesizes data on scaled down lines to data on non-scaled down lines.

12. The projector according to any one of Claims 7 or 8,
wherein the means for interpolating the image signals interpolates the image signals in response to the step of scaling down the signals by the means for scaling down the image signals.

13. The projector according to any one of Claims 7 or 8, further comprising:
a controller that sends an operational signal on which an amount of keystone correction by the means for executing keystone correction is based.
